# EUROPEAN PATENT SPECIFICATION

(11) **EP 4 179 955 B1**
(45) Date of publication and mention of the grant of the patent: **11.09.2024**
(21) Application number: 20959729.3
(22) Date of filing: 27.10.2020
(51) Int. Cl.: A61B 1/045, A61B 3/13

(54) **SURGERY ASSISTANCE DEVICE**
OPERATIONSUNTERSTÜTZUNGSVORRICHTUNG
DISPOSITIF D'ASSISTANCE CHIRURGICALE

(43) Date of publication of application: 17.05.2023
(73) Proprietor: RIVERFIELD Inc., 1070052 Tokyo (JP)
(72) Inventor: MORIKAWA, Atsushi, Tokyo 160-0017 (JP); TADANO, Kotaro, Tokyo 160-0017 (JP)
(74) Representative: adares Patent- und Rechtsanwälte Reininger & Partner GmbB
(86) International application number: PCT/JP2020/040232
(87) International publication number: WO 2022/091211

(56) References cited:
- EP-A1- 3 437 593
- WO-A1-2017/169823
- JP-A- 2004 275 203
- JP-A- 2004 275 203
- US-A1- 2017 181 798

## Description

### Technical Field

The present invention relates to a technical field of a surgery assistance device having a function of holding an endoscope.

### Background Art

Vitreous body surgery is known which restores a retina to a normal state by sucking and removing a vitreous body within an eyeball, for example, for treatment of maculopathy, retinal detachment, or the like.

In ordinary vitreous body surgery, an operator (surgeon) observes the inside of the eyeball through the pupil of a subject (patient) by a surgical microscope or the like. There is a limit to a range in which the inside of the eyeball can be observed through the pupil. In order to bring the part that cannot be observed into a viewable range, the eyeball needs to be pressed from the outside. Such pressing may cause a pain during the surgery or inflammation after the surgery.

Accordingly, a method of using an endoscope for vitreous body surgery as illustrated in PTL 1 has been proposed.

When the endoscope is inserted into the eyeball of the subject, an image of the inside of the eyeball is displayed by display means such as a monitor. The operator can easily observe a part normally unable to be viewed from the pupil by moving the endoscope within the eyeball.

The vitreous body surgery using such an endoscope obviates a need for pressing the eyeball in observing the inside of the eyeball. A burden on the eyeball of the subject can therefore be reduced.

Further, PTL2 discloses a device comprising an arm unit having arms, and a configuration in which an intermediate part of an endoscope is detachably attached to a grip part at a distal end of the arm unit. The device comprises a camera control unit performing drive control of a camera head and a display on TV monitor. The device can further contain an operating unit for rotating the endoscope captured image.

### Citation List

### Patent Literature

PTL 1: JP 2005-304633 A
PTL 2: JP 2004 275203 A

### Summary of Invention

### Technical Problem

The vitreous body surgery is performed by inserting treatment instruments such as a vitreous body cutter, forceps, and an injector of a perfusate or the like into the eyeball. The operator performs surgery on the inside of the eyeball while checking an endoscope captured image based on imaging by the endoscope on a monitor.

Here, when the imaging direction of the endoscope is shifted for positional adjustment or the like, a twist about a longitudinal axis may occur in the endoscope. When the endoscope images the inside of the eyeball in a state in which such a twist has occurred, the endoscope captured image whose upper and lower sides and left and right sides do not match the direction in which the operator is performing the surgery within the eyeball by the treatment instruments is displayed on a liquid crystal.

The display of such an endoscope captured image may cause a feeling of strangeness to the operator and may hinder smooth proceeding with the surgery.

It is accordingly an object of the present invention to display an endoscope captured image reflecting an intention of the operator.

### Solution to Problem

The object is solved by a surgery assistance device according to claim 1. A surgery assistance device according to the present invention includes an arm unit including a holder for holding an endoscope and configured to adjust a position of the endoscope in a state in which the holder holds the endoscope, a display control section configured to perform display control of an endoscope captured image that is obtained by the endoscope held by the holder, and an operating unit for rotating the endoscope captured image. The surgery assistance device further includes a computation and control unit configured to obtain image rotational angle data indicating a rotational direction and a rotational angle of the captured image data from the endoscope, to generate image data of the endoscope captured image on the basis of the captured image data from the endoscope and the image rotational angle data, and when detecting an operating input from the operating unit, to update the image rotational angle data according to an operation amount of the operating input.

Consequently, the endoscope captured image reflecting an intention of the operator performing the operation is displayed.

In the surgery assistance device described above, when an imaging direction of the endoscope held by the holder is shifted, the endoscope captured image maintaining an immediately preceding rotational state may be displayed.

Consequently, the endoscope captured image at a shifted position is subjected to rotation processing based on the same angle as before the positional shift and is displayed on a monitor.

In the surgery assistance device described above, the endoscope captured image may be a captured image of an inside of an eyeball of a subject from the endoscope inserted in the eyeball, and the display control section may display the endoscope captured image and an ocular map image indicating the position of the endoscope on a three-dimensional ocular model within the same screen.

Consequently, in performing the surgery while visually checking the endoscope captured image, it is possible to check the ocular map image without moving a line of sight to another monitor.

In the surgery assistance device described above, the operating unit may be a foot pedal.

Consequently, the operator can perform rotational operation of the endoscope captured image by a foot.

### Advantageous Effect of Invention

According to the present invention, it is possible to display an endoscope captured image reflecting an intention of the operator.

### Brief Description of Drawings

FIG. 1 is a diagram schematically illustrating an example of a configuration of a surgery system in an embodiment of the present invention.
FIG. 2 is a diagram schematically illustrating a sectional configuration of an eyeball of a subject in the present embodiment.
FIG. 3 is a diagram schematically illustrating an example of a configuration of a surgery assistance device in the present embodiment.
FIG. 4 is a diagram schematically illustrating an example of a configuration of an arm distal end section of an endoscope holding device in the present embodiment.
FIG. 5 is a diagram illustrating an example of a display image displayed on a monitor in the present embodiment.
FIG. 6 is a block diagram illustrating an example of a configuration of the surgery assistance device in the present embodiment.
FIG. 7 is a flowchart illustrating an example of processing performed by a computation and control unit of the surgery assistance device in the present embodiment.
FIG. 8 is a diagram illustrating an example of an endoscope captured image in the display image displayed on the monitor in the present embodiment.
FIG. 9 is a diagram illustrating an example of an endoscope captured image in the display image displayed on the monitor in the present embodiment.

### Description of Embodiment

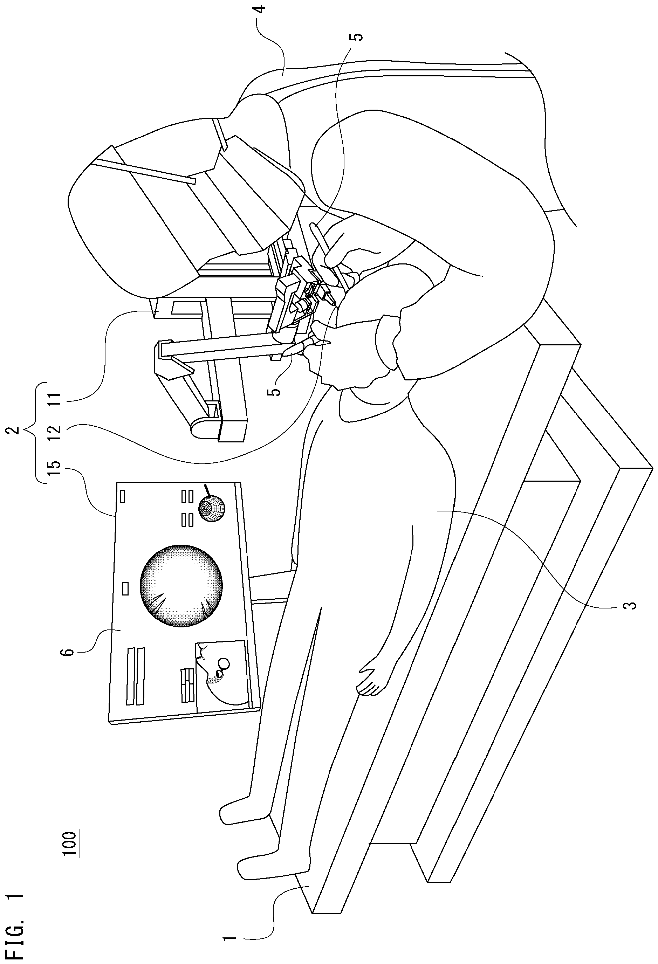

An embodiment of the present invention will be described with reference to FIGS. 1 to 9. The drawings extract and illustrate principal parts and peripheral configurations thereof recognized to be necessary for description. In addition, the drawings are schematic, and the dimensions, ratios, and the like of respective structures included in the drawings are mere examples. Hence, various changes can be made according to design or the like without departing from technical ideas of the present invention. In addition, configurations described once may be subsequently identified by the same reference numerals, and description thereof may be omitted.

The embodiment will hereinafter be described in the following order.
<1. Configuration of Surgery System>
<2. Functional Configuration of Computation and Control Unit>
<3. Example of Processing of Embodiment>
<4. Summary>

### <1. Configuration of Surgery System>

A configuration of a surgery system 100 in ocular surgery will be described.

FIG. 1 schematically illustrates an example of the configuration of the surgery system 100.

The surgery system 100 includes an operating table 1 and a surgery assistance device 2.

The operating table 1 and the surgery assistance device 2 are installed in an operating room.

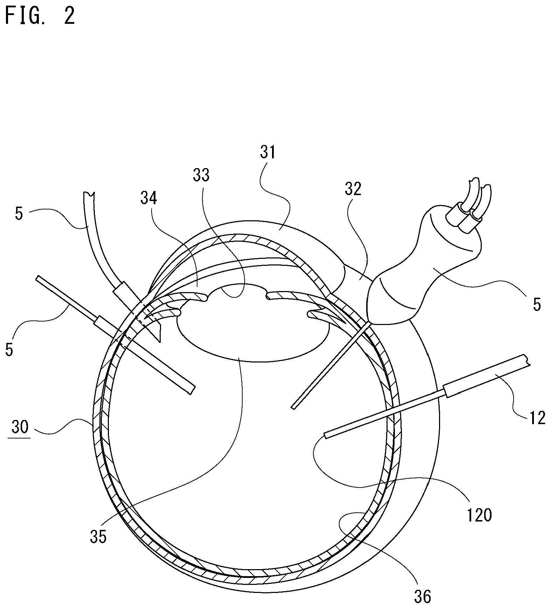

A subject (patient) 3 is laid down on his or her back on the operating table 1. An operator (surgeon) 4 is positioned on the head side of the subject 3, and performs surgery within an eyeball 30 (see FIG. 2) of the subject 3 by using various kinds of treatment instruments 5. Used as the treatment instruments 5 are, for example, a vitreous body cutter, forceps, an injector of a perfusate or the like, and the like.

FIG. 2 schematically illustrates a sectional configuration of the eyeball 30. The surface of the eyeball 30 is covered by a cornea 31 and a conjunctiva 32. An iris 34 in which a pupil 33 is formed is present in the rear of the cornea 31. A crystalline lens 35 is present in the rear of the iris 34. In addition, a retina 36 is present on the whole surface of an ocular fundus within the eyeball 30.

The operator 4, for example, inserts the treatment instruments 5 through the conjunctiva 32, and performs surgery within the eyeball 30.

The surgery assistance device 2 assists in the surgery on the eyeball 30 by the operator 4.

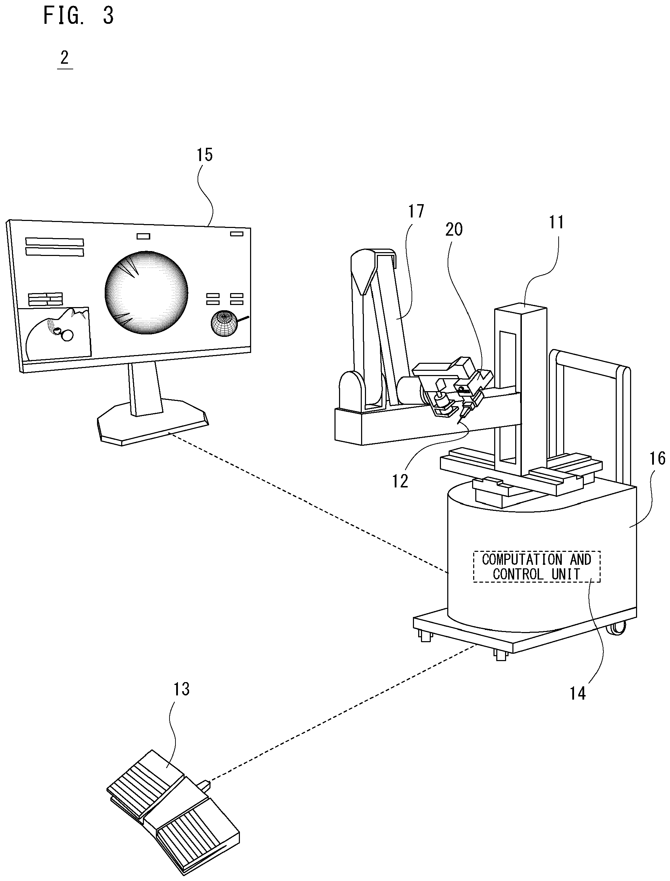

FIG. 3 schematically illustrates an example of a configuration of the surgery assistance device 2.

The surgery assistance device 2 includes an endoscope holding device 11, an endoscope 12, an operating unit 13, a computation and control unit 14, and a monitor 15.

The endoscope holding device 11 includes a base unit 16 and an arm unit 17.

The base unit 16 is mounted on the floor of the operating room or the like. The arm unit 17 is attached to the base unit 16. The arm unit 17 is pivotally supported by the base unit 16 in a rotatable manner.

The arm unit 17 includes one or a plurality of joint sections and rotary sections, and is formed as a mechanism that can move an arm distal end section 20 to a given position.

A configuration of the arm distal end section 20 will be described in the following.

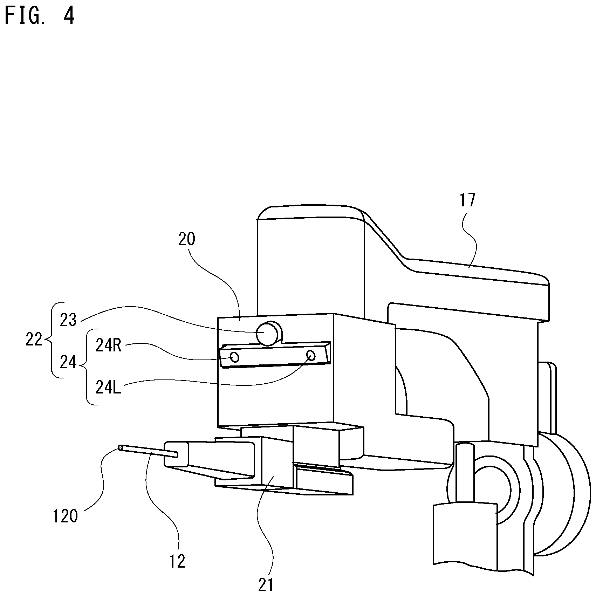

FIG. 4 schematically illustrates an example of the configuration of the arm distal end section 20.

The arm distal end section 20 includes a holder 21 for holding the endoscope 12 and a measuring unit 22 used to measure a distance to the cornea 31 of the subject 3.

The holder 21 is formed as a mechanism that allows the endoscope 12 to be attached to and detached from the holder 21. The endoscope 12 is fixed to the holder 21 by fitting the endoscope 12 into the holder 21. The endoscope 12 can be freely moved to a given position by operating the arm unit 17 in a state in which the endoscope 12 is fixed to the holder 21.

When the holder 21 holds the endoscope 12 inserted in the eyeball 30 of the subject 3, the operator 4 does not need to hold the endoscope 12 with a hand. Hence, the operator 4 can perform surgery on the eyeball 30 with both hands.

The measuring unit 22 includes an irradiating unit 23 and an imaging unit 24.

The irradiating unit 23 includes an LED (Light Emitting Diode), for example. The irradiating unit 23 outputs light that irradiates the eyeball 30 of the subject 3.

The imaging unit 24 includes imaging units 24L and 24R to be able to perform distance measurement by what is called a stereo method. The imaging units 24L and 24R are, for example, arranged at a predetermined interval from each other in the vicinity of an upper portion of the holder 21. Optical axes of the imaging units 24L and 24R are parallel with each other, and respective focal lengths of the imaging units 24L and 24R are the same value. In addition, frame periods thereof are in synchronism with each other, and frame rates thereof coincide with each other.

Captured image signals obtained by respective imaging elements of the imaging units 24L and 24R are each subjected to A/D (Analog/Digital) conversion, and are thereby converted into digital image signals (captured image data) indicating luminance values based on a predetermined gray scale in pixel units.

Distances from the imaging units 24L and 24R to the cornea 31 of the subject 3 can be measured on the basis of the captured image signals obtained by the respective imaging elements of the imaging units 24L and 24R in a state in which the irradiating unit 23 irradiates the eyeball 30.

In the measuring unit 22, relative positional relation between the irradiating unit 23 and the imaging units 24L and 24R is fixed. In addition, relative positional relation between the imaging units 24L and 24R and the above-described holder 21 is fixed. Hence, relative positional relation of the irradiating unit 23 and the imaging units 24L and 24R to the endoscope 12 is fixed by fixing the endoscope 12 to the holder 21.

Returning to FIG. 3, the endoscope 12 of the surgery assistance device 2 is inserted into the eyeball 30 in a state in which the endoscope 12 is fixed to the holder 21 (see FIG. 2). A state within the eyeball 30 is obtained by the inserted endoscope 12. Captured image signals obtained by the imaging element of the endoscope 12 are each subjected to A/D conversion, and are thereby converted into digital image signals (captured image data) indicating luminance values based on a predetermined gray scale in pixel units.

A captured image based on the captured image data from the endoscope 12 is displayed on the liquid crystal of the monitor 15.

The operating unit 13 comprehensively represents operating equipment used to perform an operation of the arm unit 17, a rotational operation of the captured image, which is displayed on the monitor 15, based on imaging by the endoscope 12, and the like. The operating unit 13 may be a foot pedal, or may be a manually operated remote operating device (remote controller) or the like. FIG. 3 illustrates a foot pedal as an example. However, as described above, the operating unit 13 is not limited to this.

Details of a method for rotational operation of the captured image based on imaging by the endoscope 12 will be described later.

The computation and control unit 14 performs various kinds of processing necessary to implement the present embodiment, such as control of operation of the arm unit 17, processing of generating various kinds of images to be displayed on the monitor 15, and processing of controlling display on the monitor 15.

The computation and control unit 14, for example, includes a microcomputer including a CPU (Central Processing Unit), a ROM (Read Only Memory), a RAM (Random Access Memory), and the like. The computation and control unit 14 is implemented by one or a plurality of microcomputers.

The computation and control unit 14 is, for example, included in the base unit 16 of the endoscope holding device 11. Incidentally, the computation and control device 14 may be included in another external apparatus.

The monitor 15 displays a display image 6 on the liquid crystal under display control from the computation and control unit 14.

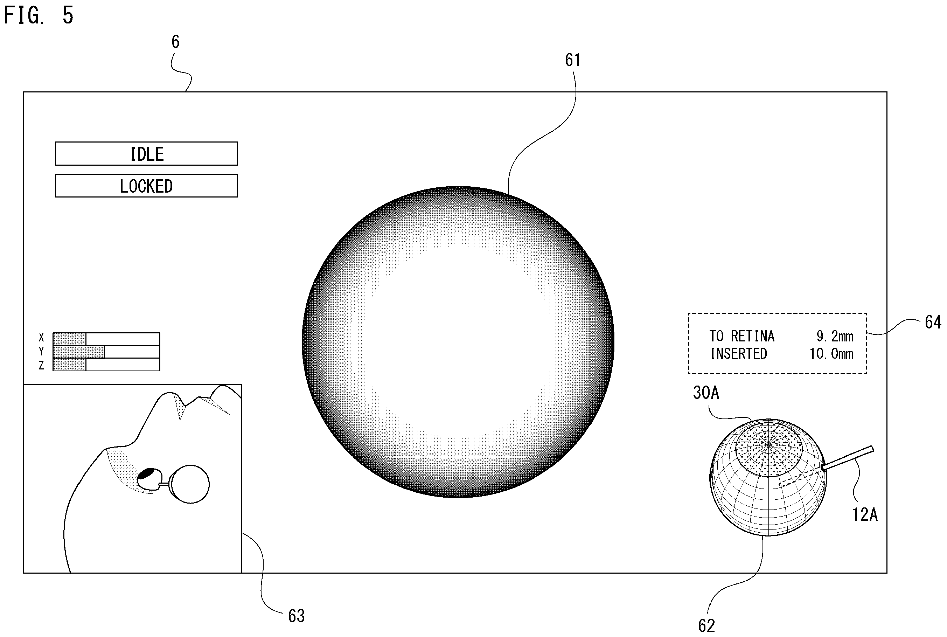

FIG. 5 illustrates an example of the display image 6 displayed on the monitor 15.

The display image 6, for example, including an endoscope captured image 61, an ocular map image 62, an endoscope viewpoint map image 63, an insertion length presenting image 64, and the like is displayed on the monitor 15. The display image 6 also includes images related to various kinds of information as required.

The endoscope captured image 61 is the captured image based on the captured image data from the endoscope 12. A state inside the eyeball 30, which is obtained by the endoscope 12, for example, is displayed as the endoscope captured image 61. The endoscope captured image 61 can be rotated by an operation using the operating unit 13. Details of a method for rotating the endoscope captured image 61 will be described later.

The ocular map image 62 illustrates positional relation between the eyeball 30 and the endoscope 12.

The ocular map image 62 displays the eyeball 30 by a three-dimensional ocular model 30A. In addition, the position of the endoscope 12 with respect to the eyeball 30 is displayed by an endoscope model 12A.

The endoscope viewpoint map image 63 displays a three-dimensional model image of the subject 3 from the viewpoint of the endoscope 12. The endoscope viewpoint map image 63 is rotated in conjunction with rotation of the endoscope captured image 61.

The insertion length presenting image 64 displays the numerical value of an insertion length of the endoscope 12 with respect to the eyeball 30 and the numerical value of a distance from an endoscope distal end portion 120 to the retina 36.

The operator 4 performs surgery on the eyeball 30 while checking the display image 6 displayed on the monitor 15.

### <2. Functional Configuration of Computation and Control Unit>

A functional configuration of the computation and control unit 14 in the surgery assistance device 2 will be described.

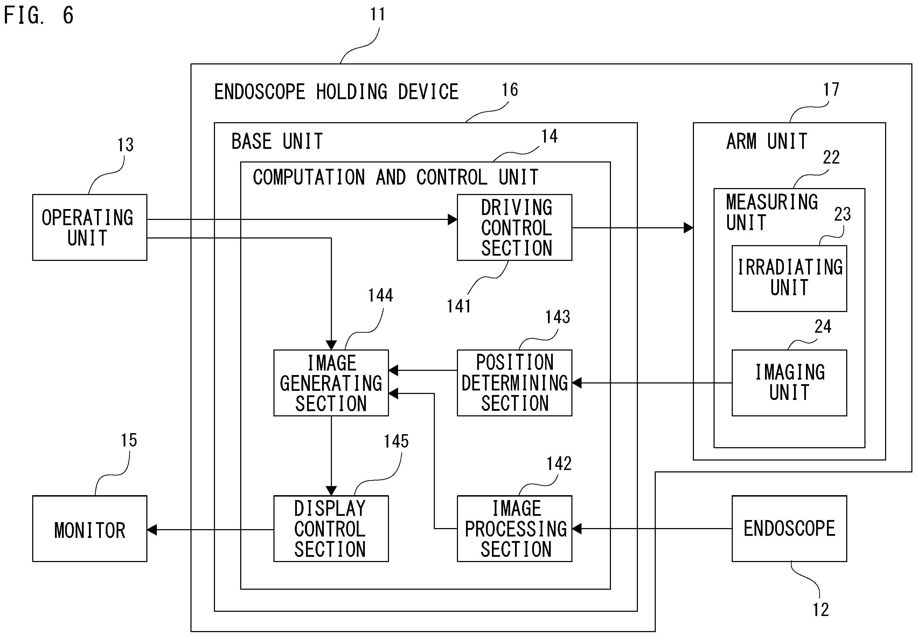

FIG. 6 illustrates, as a block diagram, an example of a configuration of the surgery assistance device 2.

The computation and control unit 14 includes a driving control section 141, an image processing section 142, a position determining section 143, an image generating section 144, and a display control section 145.

The driving control section 141 performs operation control on the joint section(s) and the rotary section(s) of the arm unit 17 of the endoscope holding device 11 on the basis of an operation signal input from the operating unit 13, for example. The driving control section 141 can move the position of the endoscope 12 fixed to the holder 21 of the arm distal end section 20 by performing operation control on the arm unit 17.

In addition, the driving control section 141 performs output control on the irradiating unit 23 and imaging control on the imaging unit 24.

The image processing section 142 subjects the image signal based on imaging by the endoscope 12 to various kinds of signal processing such as luminance signal processing, color processing, resolution conversion processing, and codec processing. The image processing section 142 outputs the image signal resulting from the various kinds of signal processing to the image generating section 144.

The position determining section 143 obtains distance information from the imaging unit 24 to the cornea 31 on the basis of the captured image signals of the eyeball 30 from the respective imaging elements of the imaging units 24L and 24R, the captured image signals being input from the imaging unit 24.

In addition, the position determining section 143 computes relative positional relation between the eyeball 30 and the endoscope distal end portion 120 on the basis of the distance information.

The position determining section 143 outputs a determination result (relative positional relation between the eyeball 30 and the endoscope distal end portion 120) to the image generating section 144.

The image generating section 144 generates the display image 6 as illustrated in FIG. 5 by using various kinds of input information from the image processing section 142, the position determining section 143, the operating unit 13, and the like. Details of a method for generating various kinds of images constituting the display image 6 will be described later.

The image generating section 144 outputs an image signal of the generated display image 6 to the display control section 145.

The display control section 145 performs control that displays the display image 6 on the monitor 15 on the basis of the image signal input from the image generating section 144.

### <3. Example of Processing of Embodiment>

Description will be made of processing performed by the computation and control unit 14 of the surgery assistance device 2 in order to implement the present embodiment. Here, suppose, as an example, that the operator 4 is performing surgery while positioned on the head side of the subject 3 (see FIG. 1).

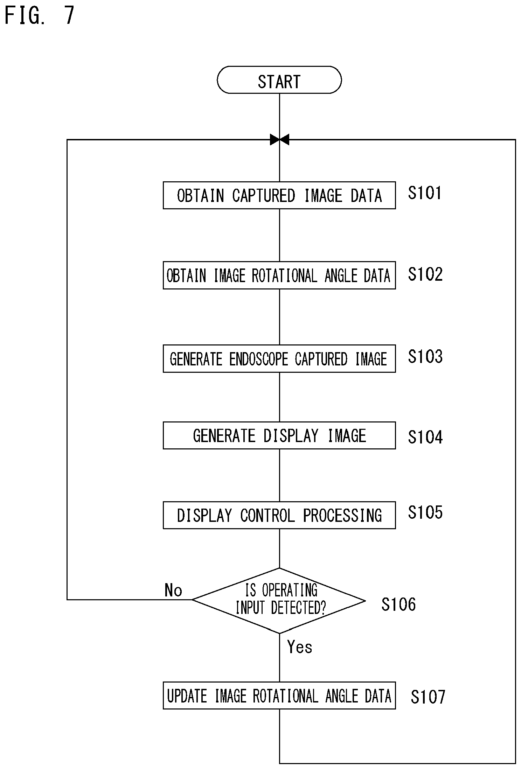

FIG. 7 is a flowchart illustrating an example of the processing performed by the computation and control unit 14. The computation and control unit 14 repeatedly performs the processing of FIG. 7 in timing of each frame of the image.

In step S101, the computation and control unit 14 obtains the captured image data input from the endoscope 12. The computation and control unit 14 stores, in an internal memory, each piece of frame image data, which is the captured image data obtained by imaging the inside of the eyeball 30 by the endoscope 12.

In step S102, the computation and control unit 14 obtains image rotational angle data.

The image rotational angle data is information indicating a rotational direction and a rotational angle of the captured image data from the endoscope 12, which is used to perform rotation processing on the captured image data.

The image rotational angle data is updated as appropriate according to operating input from the operating unit 13. However, the image rotational angle data is not updated when an imaging direction of the endoscope 12 is shifted according to operation of the arm unit 17.

During a period from a start of the processing of FIG. 7 by the computation and control unit 14 to detection of an operating input by the operating unit 13 (the period will hereinafter be described also as an initial state), the image rotational angle data is a value indicating an angle of 0° in rotation processing on the endoscope captured image.

When the computation and control unit 14 detects an operating input from the operating unit 13, the computation and control unit 14 updates the image rotational angle data according to an operation amount of the operating input. The rotational direction is indicated by a positive or negative angle of the updated image rotational angle data, for example. Details of update processing on the image rotational angle data will be described later.

In the step S 103, the computation and control unit 14 generates image data of the endoscope captured image 61 on the basis of the captured image data from the endoscope 12 and the image rotational angle data.

In this case, the computation and control unit 14 at least generates the image data of the endoscope captured image 61 obtained by rotating the captured image data from the endoscope 12 on the basis of the direction and the angle based on the image rotational angle data.

In addition, in a case of the initial state, a rotational angle of 0° is indicated as image rotational angle data, and therefore the computation and control unit 14 generates the image data as the endoscope captured image 61 without rotating the captured image data from the endoscope 12.

In step S104, the computation and control unit 14 generates image data of the display image 6.

The computation and control unit 14 generates the image data of the display image 6 by synthesizing the endoscope captured image 61, the ocular map image 62, the endoscope viewpoint map image 63, the insertion length presenting image 64, and image data of other necessary images.

Here, description will be made of an example of a method for generating image data of various kinds of images other than the endoscope captured image 61 described above.

### - Ocular Map Image 62

The computation and control unit 14 performs various kinds of image analysis processing such, for example, as recognition of light spots of light 25 from the irradiating unit 23, which appear on the eyeball 30 or the cornea 31 on the basis of the pair of captured image data as respective frames obtained by imaging the eyeball 30 by the imaging units 24L and 24R.

For the pair of captured image data (stereo images), the computation and control unit 14 computes an imaging distance, which is a distance from the imaging unit 24 to the cornea 31, by a principle of triangulation from an amount of offset between the positions of the light spots appearing on the cornea 31.

The computation and control unit 14 determines positional relation between the imaging unit 24 and the eyeball 30 on the basis of the computed imaging distance, and determines the positional relation between the endoscope 12 (endoscope distal end portion 120), whose relative positional relation to the imaging unit 24 is fixed, and the eyeball 30.

The computation and control unit 14 generates image data of the ocular map image 62 illustrating the determined positional relation between the eyeball 30 and the endoscope 12 (endoscope distal end portion 120) as positional relation between the three-dimensional ocular model 30A and the endoscope model 12A.

### - Endoscope Viewpoint Map Image 63

The computation and control unit 14 generates image data of the endoscope viewpoint map image 63 displaying a three-dimensional model image of the subject 3 from the viewpoint of the endoscope 12.

Preset three-dimensional model data of a head portion of a human is used as the three-dimensional model of the subject 3. A value indicating the angle of the head portion of the subject 3 with respect to the endoscope 12 is set as head portion angle data in advance on an assumption that the subject 3 is laid down on his or her back on the operating table 1 and an installation angle of the endoscope holding device 11 with respect to the operating table 1 is defined.

The computation and control unit 14 generates the three-dimensional model image including the three-dimensional ocular model image on the basis of the three-dimensional model data and the head portion angle data.

Then, the computation and control unit 14 generates the image data of the endoscope viewpoint map image 63 by synthesizing the three-dimensional model image and the endoscope model image on the basis of the positional relation between the eyeball 30 and the endoscope 12.

### - Insertion Length Presenting Image 64

The computation and control unit 14 computes information regarding the insertion length of the endoscope 12 in the eyeball 30 and information regarding the distance from the endoscope distal end portion 120 to the retina 36 of the subject 3 from the determined positional relation between the eyeball 30 and the endoscope 12 (endoscope distal end portion 120).

Then, the computation and control unit 14 generates the image data of the insertion length presenting image 64 on the basis of these pieces of information.

The image data of the various kinds of images necessary to generate the image data of the display image 6 is generated by the method described above.

In the following step S105, the computation and control unit 14 performs display control for displaying the display image 6 on the liquid crystal of the monitor 15. The display image 6 as illustrated in FIG. 5 is thereby displayed within the same screen of the monitor 15.

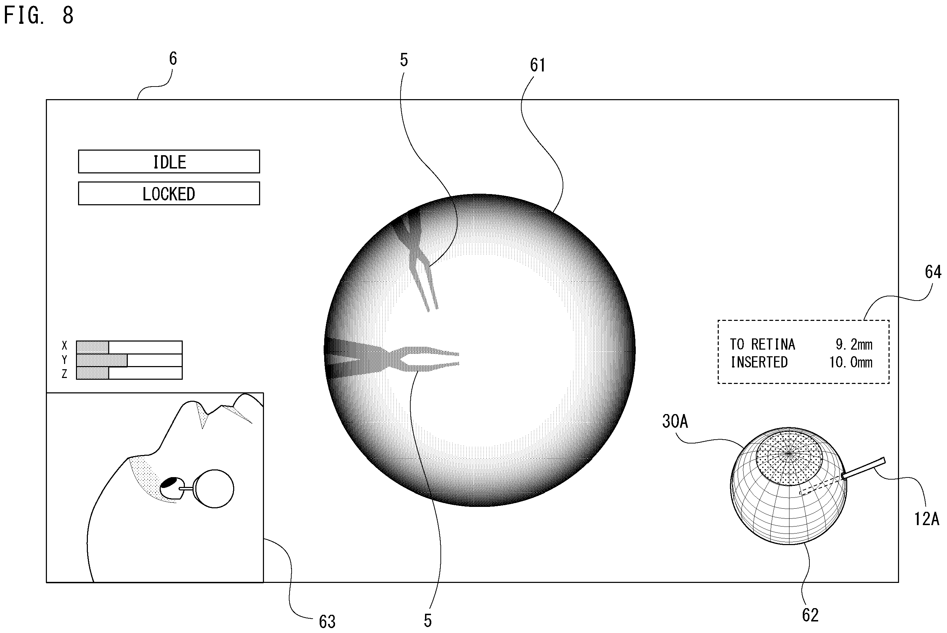

Incidentally, during the surgery on the eyeball 30 by the operator 4, treatment instruments 5 may appear in the endoscope captured image 61, as illustrated in FIG. 8. In this case, as is clear from the display positions of the treatment instruments 5, the upper and lower sides and left and right sides of the endoscope captured image 61 do not match a direction in which the operator 4 illustrated in FIG. 1 performs the surgery on the inside of the eyeball 30.

This is caused by the occurrence of a twist in the endoscope 12 about a longitudinal axis at a time of adjustment of the position of the endoscope 12 inserted into the eyeball 30. When the endoscope 12 images the inside of the eyeball 30 in a state in which such a twist has occurred, the endoscope captured image 61 whose upper and lower sides and left and right sides do not match the direction in which the operator 4 is performing the surgery is displayed on the monitor 15.

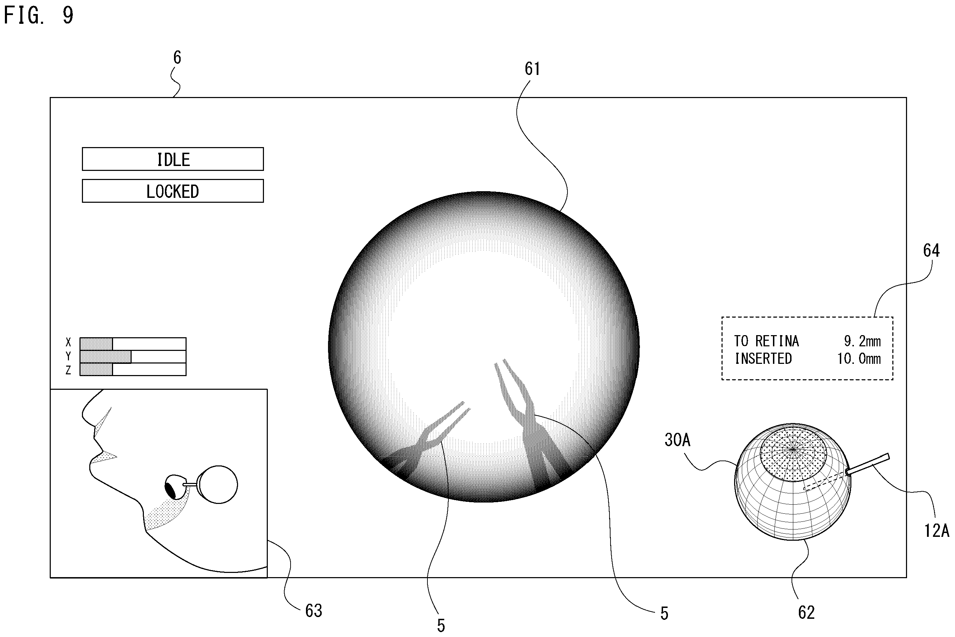

In such a case, the operator 4 needs to rotate the endoscope captured image 61 to an easily viewable position (for example, a position illustrated in FIG. 9) by operating the operating unit 13 in order to proceed with the surgery smoothly.

The computation and control unit 14 therefore performs the processing of steps S106 and S 107.

In step S106, the computation and control unit 14 determines whether or not an operating input from the operating unit 13 is detected. An operating input is, for example, performed by the operator 4 by operating the foot pedal with a foot.

When no operating input is detected in step S106, the computation and control unit 14 returns the processing to step S101, and subsequently performs similar processing.

That is, in the initial state, the endoscope captured image 61 in a non-rotated state are displayed on the monitor 15.

When an operating input is detected in step S106, on the other hand, the computation and control unit 14 advances the processing from step S106 to S107.

In step S107, the computation and control unit 14 performs update processing on the image rotational angle data.

The computation and control unit 14 updates the rotational direction and the rotational angle as the image rotational angle data on the basis of the operating input from the operating unit 13.

After performing the processing of step S107, the computation and control unit 14 returns the processing to step S101. Then, the computation and control unit 14 performs the processing from step S101 to S103, and thereby generates the image data of the endoscope captured image 61 obtained by rotating the captured image data from the endoscope 12 on the basis of the updated image rotational angle data.

When such processing is performed, the endoscope captured image 61 displayed on the monitor 15 can continue to be rotated according to the operation of the operating unit 13 without causing a positional displacement of the endoscope 12 within the eyeball 30. For example, the endoscope captured image 61 can be rotated from a position illustrated in FIG. 8 to a position illustrated in FIG. 9.

In the following step S104, the computation and control unit 14 generates the image data of the display image 6 including the endoscope captured image 61 as described above.

In step S105, the computation and control unit 14 performs display control for displaying the display image 6 on the liquid crystal of the monitor 15. The endoscope captured image 61 rotated according to the operation of the operating unit 13 by the operator 4 is displayed on the monitor 15.

While the operator 4 continues operating the operating unit 13, the computation and control unit 14 continues performing the processing from step S101 to step S107.

The operator 4, for example, continues operating the operating unit 13 to rotate the endoscope captured image 61 until the treatment instruments 5 appearing in the endoscope captured image 61 are located at positions that facilitate the surgery.

When the operator 4 ends the operation of the operating unit 13, the computation and control unit 14 determines that no operating input is detected in step S106. In this case, the computation and control unit 14 returns the processing from step S106 to S101, and thereafter repeatedly performs the processing from step S101 to step S106 until an operating input is detected in step S106.

At this time, the computation and control unit 14 generates the image data of the endoscope captured image 61 on the basis of the last updated image rotational angle data, and performs display control on the monitor 15. Thus, the endoscope captured image 61 is displayed on the monitor 15 in a state in which the rotational angle at a time point of a stop of the rotation is maintained.

Incidentally, the computation and control unit 14 does not update the image rotational angle data even when the endoscope 12 held by the holder 21 is moved by operation control on the arm unit 17 to shift the imaging direction of the endoscope 12.

Hence, on the monitor 15, the endoscope captured image 61 obtained by shifting only the imaging direction is displayed in a state in which the rotation based on the last updated image rotational angle data is maintained.

Hence, the operator 4 does not need to rotate the endoscope captured image 61 again by operating the operating unit 13 when the imaging direction of the endoscope 12 is shifted.

When the computation and control unit 14 detects an operating input again in step S106 while repeatedly performing the processing from step S101 to step S106, the computation and control unit 14 advances the processing in order of steps S107 and S101, and thereafter performs similar processing.

### <4. Summary>

The surgery assistance device 2 in the embodiment of the present invention includes the arm unit 17 including the holder 21 for holding the endoscope 12 and configured to adjust the position of the endoscope 12 in a state in which the holder 21 holds the endoscope 12, the display control section 145 configured to perform display control of the endoscope captured image 61 that is obtained by the endoscope 12 held by the holder 21, and the operating unit 13 for rotating the endoscope captured image 61. The displayed endoscope captured image 61 is rotated according to operation of the operating unit 13 without a positional displacement of the endoscope 12 being caused (see FIG. 6, FIG. 7, and the like).

Consequently, the endoscope captured image 61 reflecting an intention of the operator 4 operating the operating unit 13 is displayed.

Hence, even when the endoscope captured image 61 whose upper and lower sides and left and right sides do not match the direction in which the operator 4 is performing the surgery within the eyeball 30 is displayed on the monitor 15 (see FIG. 8), the endoscope captured image 61 can be adjusted, by an operation, to a position (see FIG. 9) that facilitates the operator 4 performing the surgery. It is therefore possible to facilitate smooth proceeding with the surgery.

In the surgery assistance device 2 in the present embodiment, when the imaging direction of the endoscope 12 held by the holder 21 is shifted, the endoscope captured image 61 maintaining an immediately preceding rotational state is displayed (see S103 in FIG. 7 and the like).

Consequently, the endoscope captured image 61 at a shifted position is subjected to rotation processing based on the same angle as before the positional shift, and is displayed on the monitor 15.

Hence, the endoscope captured image 61 reflecting an intention of the operator 4 to maintain the rotational state can be displayed. In addition, the operator 4 does not need to operate the operating unit 13 each time the position of the endoscope 12 is shifted. Thus, smooth proceeding with the surgery can be facilitated.

In the surgery assistance device 2 in the present embodiment, the endoscope captured image 61 is a captured image of the inside of the eyeball 30 from the endoscope 12 inserted in the eyeball 30 of the subject 3, and the display control section 145 displays the endoscope captured image 61 and the ocular map image 62 indicating the position of the endoscope 12 on the three-dimensional ocular model 30A within the same screen (see S105 in FIG. 7 and the like).

Consequently, by visually checking the monitor 15 displaying the endoscope captured image 61, it is possible also to grasp the position of the endoscope 12 inserted in the eyeball 30 of the subject 3.

Hence, the operator 4 can check the position of the endoscope 12 inserted within the eyeball 30 without moving a viewpoint from the display screen 6 displaying video of the inside of the eyeball 30. It is therefore possible to proceed with the surgery on the eyeball 30 more smoothly.

In the surgery assistance device 2 in the present embodiment, the operating unit 13 is a foot pedal (see FIG. 3 and the like).

Consequently, the operator 4 can perform rotational operation of the endoscope captured image 61 by a foot.

Hence, the endoscope captured image 61 reflecting an intention of the operator 4 can be displayed even when both hands of the operator 4 are full during the surgery. In other words, the operator 4 can use both hands freely even while performing a rotational operation of the endoscope captured image 61. It is therefore possible to proceed with the surgery smoothly.

It is to be noted that, while an example of an intraocular endoscope has been described as an example of the endoscope 12 in the present embodiment, the endoscope 12 is not limited to the intraocular endoscope. For example, it is possible to apply various endoscopes such as a thoracoscope inserted after an incision between ribs of the subject 3 and a laparoscope inserted after an incision in an abdomen.

### Reference Signs List

2: Surgery assistance device
3: Subject
12: Endoscope
13: Operating unit
17: Arm unit
21: Holder
30: Eyeball
30A: Three-dimensional ocular model
61: Endoscope captured image
62: Ocular map image
145: Display control section

## Claims

1. A surgery assistance device (2) comprising:
an arm unit (17) including a holder (21) for holding an endoscope (12) and configured to adjust a position of the (12) endoscope in a state in which the holder (21) holds the endoscope (12);
a display control section (145) configured to perform display control of an endoscope captured image (61) that is obtained by the endoscope (12) held by the holder (21); and
an operating unit (13) configured to provide an operating input from an operator for rotating the endoscope captured image (61),
a computation and control unit (14) which is configured to obtain image rotational angle data indicating a rotational direction and a rotational angle of the captured image data from the endoscope (12), to generate image data of the endoscope captured image (61) on the basis of the captured image data from the endoscope (12) and the image rotational angle data, and when detecting the operating input from the operating unit (13), to update the image rotational angle data according to an operation amount of the operating input.

2. The surgery assistance device (2) according to claim 1, wherein,
when an imaging direction of the endoscope (12) held by the holder (21) is shifted the display control section (145) is configured to display the endoscope captured image (61) maintaining an immediately preceding rotational state.

3. The surgery assistance device (2) according to claim 1 or 2, wherein
the endoscope captured image (61) is a captured image of an inside of an eyeball (30) of a subject (3) from the endoscope (12) inserted in the eyeball (30), and
the display control section (145) is configured to display
the endoscope captured image (61) and an ocular map image (62) indicating the position of the endoscope (12) on a three-dimensional ocular model (30A) within a same screen.

4. The surgery assistance device (2) according to any one of claims 1 to 3, wherein the operating unit (13) is a foot pedal.

## Patentansprüche

1. Chirurgie-Assistenzvorrichtung (2), enthaltend:
eine Armeinheit (17), die einen Halter (21) zum Halten eines Endoskops (12) enthält und zum Einstellen einer Position des (12) Endoskops in einem Zustand konfiguriert ist, in dem der Halter (21) das Endoskop (12) hält;
einen Anzeigesteuerabschnitt (145), der konfiguriert ist, eine Anzeigesteuerung eines Endoskop-erfassten Bildes (61) durchzuführen, das von dem vom Halter (21) gehaltenen Endoskop (12) erhalten wird; und
eine Bedieneinheit (13), die zum Bereitstellen einer Bedieneingabe von einem Bediener zum Drehen des Endoskop-erfassten Bildes (61) konfiguriert ist,
eine Rechen- und Steuereinheit (14), die konfiguriert ist, Bilddrehwinkeldaten zu erhalten, die eine Drehrichtung und einen Drehwinkel der erfassten Bilddaten vom Endoskop (12) anzugeben, um Bilddaten des Endoskop-erfassten Bildes (61) auf der Basis der vom Endoskop (12) erfassten Bilddaten und der Bilddrehwinkeldaten zu erzeugen, und wenn die Bedieneingabe von der Bedieneinheit (13) detektiert wird, die Bilddrehwinkeldaten entsprechend eines Bedienmaßes des Bedieneingabe zu aktualisieren.

2. Chirurgie-Assistenzvorrichtung (2) nach Anspruch 1, wobei
wenn eine Abbildungsrichtung des Endoskops (12), das von dem Halter (21) gehalten wird, verschoben wird, der Anzeigesteuerabschnitt (145) konfiguriert ist, das Endoskop-erfasste Bild (61) unter Beibehalten eines unmittelbar vorhergehenden Drehzustands anzuzeigen.

3. Chirurgie-Assistenzvorrichtung (2) nach Anspruch 1 oder 2, wobei
das Endoskop-erfasste Bild (61) ein erfasstes Bild einer Innenseite eines Augapfels (30) eines Subjekts (3) von dem in den Augapfel (30) eingeführten Endoskop (12) ist, und
der Anzeigesteuerabschnitt (145) konfiguriert ist, das Endoskop-erfasste Bild (61) und ein Okularkartenbild (62) anzuzeigen, das die Position des Endoskops (12) auf einem dreidimensionalen Okularmodell (30A) auf einem gleichen Bildschirms anzeigt.

4. Chirurgie-Assistenzvorrichtung (2) nach einem der Ansprüche 1 bis 3, wobei die Bedieneinheit (13) ein Fußpedal ist.

## Revendications

1. Un dispositif d'assistance chirurgicale (2) comprenant :
une unité de bras (17) comprenant un support (21) pour tenir un endoscope (12) et configurée pour ajuster une position de l'endoscope (12) dans un état dans lequel le support (21) tient l'endoscope (12) ;
une section de commande d'affichage (145) configurée pour effectuer un contrôle d'affichage d'une image capturée par un endoscope (61) obtenue par l'endoscope (12) tenu par le support (21) ; et
une unité opérationnelle (13) configurée fournir une entrée opérationnelle d'un opérateur pour tourner l'image capturée par un endoscope (61),
une unité de calcul et de commande (14) qui est configurée pour obtenir des données d'angle de rotation d'image indiquant une direction de rotation et un angle de rotation des données d'image capturée à partir de l'endoscope (12), pour générer des données d'image de l'image capturée par un endoscope (61) sur la base des données d'image capturées à partir de l'endoscope (12) et des données d'angle de rotation de l'image, et lors de la détection d'une entrée opérationnelle de l'unité opérationnelle (13), mettre à jour les données d'angle de rotation de l'image en fonction d'une quantité d'opération de l'entrée opérationnelle.

2. Le dispositif d'assistance chirurgicale (2) selon la revendication 1, dans lequel :
lorsqu'une direction d'imagerie de l'endoscope (12) tenu par le support (21) est déplacée, la section de commande d'affichage (145) est configurée pour afficher l'image capturée par un endoscope (61) en maintenant un état de rotation immédiatement précédent.

3. Le dispositif d'assistance chirurgicale (2) selon la revendication 1 ou 2, dans lequel
l'image capturée par un endoscope (61) est une image capturée de l'intérieur d'un globe oculaire (30) d'un sujet (3) à partir de l'endoscope (12) inséré dans le globe oculaire (30), et
la section de commande d'affichage (145) est configurée pour afficher l'image capturée par un endoscope (61) et une image de carte oculaire (62) indiquant la position de l'endoscope (12) sur un modèle oculaire tridimensionnel (30A) dans un même écran.

4. Le dispositif d'assistance chirurgicale (2) selon l'une quelconque des revendications 1 à 3, dans lequel
l'unité opérationnelle (13) est une pédale de pied.
